# EUROPEAN PATENT SPECIFICATION

(11) **EP 3 377 479 B1**
(45) Date of publication and mention of the grant of the patent: **29.01.2020**
(21) Application number: 16797944.2
(22) Date of filing: 18.11.2016
(51) Int. Cl.: C07D 401/04, A61K 31/445, A61P 37/08

(54) **SUPERSATURATED COMPOSITIONS OF BENZIMIDAZOLE COMPOUNDS**
ÜBERSÄTTIGTE ZUSAMMENSETZUNGEN VON BENZIMIDAZOLVERBINDUNGEN
COMPOSITIONS SURSATURÉES DE COMPOSÉS DE BENZIMIDAZOLE

(30) Priority: 20.11.2015 EP 15382574
(43) Date of publication of application: 26.09.2018
(73) Proprietor: FAES FARMA, S.A., 48940 Leioa - Vizcaya (ES)
(72) Inventor: HERNÁNDEZ HERRERO, Gonzalo, E-48940 Leioa-Vizcaya (ES); RUBIO ROYO, Víctor, E-48940 Leioa- Vizcaya (ES); GARCÍA DOMÍNGUEZ, Neftalí, E-48940 Leioa-Vizcaya (ES); CANAL MORI, Gonzalo, E-48940 Leioa-Vizcaya (ES); TESSON, Nicolas, E-08028 Barcelona (ES)
(74) Representative: ABG Intellectual Property Law, S.L.
(86) International application number: PCT/EP2016/078154
(87) International publication number: WO 2017/085265

(56) References cited:
- WO-A1-03/089425
- WO-A2-2008/054609
- CN-A- 103 351 380

## Description

### Field of the Invention

The present invention relates to supersaturated aqueous solutions of benzimidazole compounds and their use as antihistamine and antiallergic compositions. The invention also relates to the use of organic carboxylic acids to increase the aqueous solubility of benzimidazole compounds.

### Background of the Invention

It has long been known that histamine plays a very important role in allergic-type diseases, such as allergic rhinitis, conjunctivitis, rhinoconjunctivitis, dermatitis, urticaria and asthma. Antihistaminic compounds acting at the H₁-receptor histamine level are useful for treating such conditions.

Documents EP 0818454 A1 and EP 0580541 A1 disclose benzimidazole compounds with selective H₁ antihistaminic activity and devoid of arrhythmogenic effects. Patent application EP14382576.8 also discloses benzimidazole compounds having potent selective H₁ antihistaminic activity, lacking activity on the central nervous system and on the cardiovascular system.

A particular compound with the above properties is 2-[4-(2-{4-[1-(2-Ethoxyethyl)-1H-benzimidazol-2-yl]-1-piperidinyl}ethyl)phenyl]-2-methylpropanoic acid, also known as bilastine, having formula: 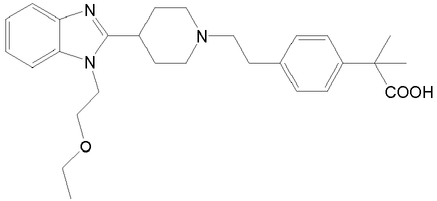 and developed by Faes Farma, Spain, is a H₁ antagonist benzimidazole compound with no sedative side effects, no cardiotoxic effects, and no hepatic metabolism. In addition, bilastine has proved to be effective for the symptomatic treatment of allergic rhinoconjunctivitis and urticaria.

The above benzimidazole compounds with selective H₁ antihistaminic activity present low solubility in water, which impedes the development of pharmaceutically acceptable means of administering said compounds in liquid form. For example, the solubility of bilastine in the pH range 5-8 is around 500 µg/mL.

Therefore, there is a need in the art to provide a method for improving the solubility in water of said benzimidazole compounds with selective H₁ antihistaminic activity. The present invention addresses such concern.

### Summary of the Invention

The applicant has surprisingly found that organic carboxylic acids selected from the group consisting of substituted or unsubstituted aliphatic C₃-C₈ α,ω-dicarboxylic acids and substituted or unsubstituted aliphatic C₂-C₆ monocarboxylic acids highly improve the aqueous solubility of bilastine. Particularly, it has been found that such organic carboxylic acids allow obtaining an aqueous solubility of bilastine above the pH-dependent solubility. Supersaturated aqueous solutions of bilastine can be obtained wherein the solubility is maintained over time.

Therefore, in a first aspect the invention is directed to a supersaturated aqueous solution of bilastine, comprising an organic carboxylic acid selected from glutaric acid, citric acid, α-cetoglutaric acid, tartaric acid and mixtures thereof.

In another aspect, the invention refers to the use of an organic carboxylic acid selected from glutaric acid, citric acid, α-cetoglutaric acid, tartaric acid, acetic acid, propionic acid and mixtures thereof to increase the aqueous solubility of bilastine as defined above.

In a further aspect, the invention is directed to a method for preparing a supersaturated aqueous solution of bilastine comprising
(a) preparing a slurry of bilastine and the organic carboxylic acid in water, and
(b) heating the slurry of step (a) to obtain a solution, and
(c) cooling the solution.

In another aspect, the invention is directed to a method for preparing a supersaturated aqueous solution of bilastine comprising
(a) preparing a co-crystal of bilastine and the organic carboxylic acid, and
(b) dissolving the co-crystal of step (a) in water or an aqueous solution.

In a further aspect, the invention refers to a pharmaceutical composition comprising a supersaturated aqueous solution of bilastine and at least one pharmaceutically acceptable excipient.

In another aspect, the invention refers to the supersaturated aqueous solution or the pharmaceutical composition described herein for use as a medicament. Preferably, for use in the treatment or prevention of a disorder or disease susceptible to amelioration by antagonism of H₁ histamine receptor such as allergic disease or disorder.

Another aspect of this invention refers to the supersaturated aqueous solution or the pharmaceutical composition described herein for use in the prevention and/or treatment of an allergic disease or disorder, such as rhinitis, conjunctivitis, rhinoconjuntivitis, dermatitis, urticaria and asthma.

### Brief description of the drawings

**Figure 1****.** Graph showing the pH-dependent solubility (mg/ml) of bilastine.
**Figure 2****.** Characterization of bilastine BLN(I) - glutaric acid cocrystal GL(I): Fig. 2 a) XRPD; Fig. 2 b) ¹H-RMN in DMSO; Fig. 2 c) TGA; Fig. 2 d) Solubility comparison BLN(I) vs GL(I).
**Figure 3****.** Characterization of bilastine BLN(I) - glutaric acid cocrystal GL(IV): Fig. 3 a) XRPD; Fig. 3 b) ¹H-RMN in DMSO; Fig. 3 c) TGA; Fig. 3 d) Solubility comparison BLN(I) vs GL(IV).
**Figure 4****.** Characterization of bilastine BLN(I) - glutaric acid cocrystal GL(V): Fig. 4 a) XRPD; Fig. 4 b) ¹H-RMN in DMSO; Fig. 4 c) TGA; Fig. 4 d) Solubility comparison BLN(I) vs GL(V).

### Detailed Description of the Invention

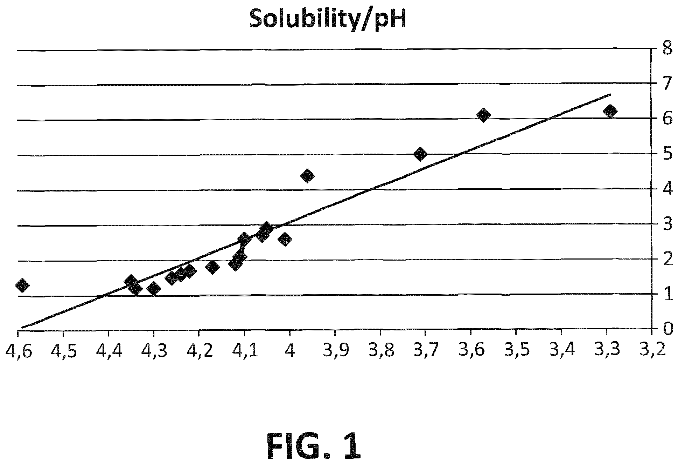

Bilastine presents a pH-dependent solubility in water. Figure 1 shows the aqueous pH-dependent solubility of bilastine at a pH range between 3.3 and 4.6. It has been surprisingly found that organic carboxylic acids selected from the group consisting of substituted or unsubstituted aliphatic C₃-C₈ α,ω-dicarboxylic acids and substituted or unsubstituted aliphatic C₂-C₆ monocarboxylic acids allow obtaining an aqueous solubility of bilastine above the pH-dependent solubility. In particular, supersaturated aqueous solutions of bilastine can be obtained wherein the solubility is maintained over time.

Within the scope of the invention, the term "saturated solution" means a solution containing a concentration of bilastine that is equal to the maximum amount of bilastine that can be dissolved at a specific temperature, typically set at 20°C, and pH (the so-called "saturation concentration").
at a specific temperature and pH. That is, a supersaturated solution is a solution containing a concentration of bilastine that is higher than its saturation concentration and wherein the full amount of bilastine is still completely dissolved. Therefore, a supersaturated aqueous solution of bilastine and an organic carboxylic acid as defined herein means an aqueous solution that has a concentration of bilastine greater than the one that would be present in a saturated aqueous solution of bilastine at a given temperature, typically 20°C, and pH.

Bilastine presents a pH-dependent solubility. Therefore, the supersaturated aqueous solution of the invention allows solubilizing an amount of bilastine that is above its pH-dependent solubility.

Supersaturated solutions are expected to be thermodynamically unstable leading to precipitation or crystallization of bilastine. However, it has been surprisingly found that the solubility of bilastine in the supersaturated aqueous solutions of the invention is maintained over time. They are stable supersaturated solutions.

In a particular embodiment, the supersaturated aqueous solution of the invention is stable for at least 12 h, preferably for at least 24 h under standard ambient conditions. This can be determined, for example, by measuring the solubility of bilastine by HPLC after said time.

The term "alkyl" refers to a linear or branched saturated hydrocarbon chain radical consisting of carbon and hydrogen atoms and which is attached to the rest of the molecule by a single bond. Particularly, the term "C₁₋₆ alkyl" refers to an alkyl having between 1 and 6 carbon atoms. The term "C₁₋₃ alkyl" refers to an alkyl having 1, 2 or 3 carbon atoms. Alkyl groups include for example and in a non-limiting sense, methyl, ethyl, n-propyl, i-propyl, n-butyl, t-butyl, n-pentyl, etc. Preferably "alkyl" refers to methyl or ethyl.

As understood in this technical area, there can be a certain degree of substitution on the previously defined radicals. The references to substituted groups indicate that the specified radical can be substituted in one or more available positions by one or more substituents.

### Bilastine

The aqueous pharmaceutical composition of the invention comprises bilastine of formula,

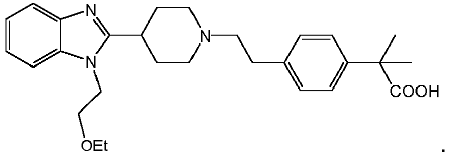

This compound is 2-[4-(2-{4-[1-(2-Ethoxyethyl)-1H-benzimidazol-2-yl]-1-piperidinyl}ethyl)phenyl]-2-methylpropanoic acid, also known as bilastine. The synthesis of bilastine has been described in EP 0818454 A1.

### Organic carboxylic acid

The organic carboxylic acid according to the invention is selected from the group consisting of substituted or unsubstituted aliphatic C₃-C₈ α,ω-dicarboxylic acids and substituted or unsubstituted aliphatic C₁-C₆ carboxylic acids.

"Aliphatic C₃-C₈ α,ω-dicarboxylic acid" refers to an organic compound containing two carboxyl functional groups at the two ends of a C₃-C₈ saturated or unsaturated aliphatic chain, preferably a C₄-C₆ saturated or unsaturated aliphatic chain ("aliphatic C₄-C₆ α,ω-dicarboxylic acid"). That is, it is a compound of formula HOOC-R'-COOH, wherein R' is the saturated or unsaturated C₁-C₆, preferably a C₂-C₄, aliphatic chain. Preferably the R' group is a saturated chain.

In a particular embodiment, the R' group is an unsubstituted saturated chain, that is R' is -(CH₂)ₙ- wherein n is 1, 2, 3, 4, 5 or 6, preferably n is 2, 3 or 4.

The aliphatic C₃-C₈ α,ω-dicarboxylic acids can be substituted or unsubstituted. The substituents may be selected from the group consisting of -OH, -COOH and =O. Preferably, the aliphatic C₃-C₈ α,ω-dicarboxylic acid has one, two or three substituents selected from -OH, -COOH and =O, more preferably one or two.

In an embodiment, the aliphatic C₃-C₈ α,ω-dicarboxylic acid is an aliphatic C₄ α,ω-dicarboxylic acid, aliphatic C₅ α,ω-dicarboxylic acid, aliphatic C₆ α,ω-dicarboxylic acid or an aliphatic C₇ α,ω-dicarboxylic acid.

In a particular embodiment, the aliphatic C₃-C₈ α,ω-dicarboxylic acid is selected from glutaric acid, citric acid, α-cetoglutaric acid, tartaric acid, malic acid, adipic acid and succinic acid. Preferably, it is selected from glutaric acid, citric acid, tartaric acid and α-cetoglutaric acid. More preferably, it is glutaric acid.

The term "aliphatic C₂-C₆ monocarboxylic acid" refers to a saturated or unsaturated aliphatic chain containing one carboxyl functional group. The carboxyl functional group is preferably present at one end of the chain, having a molecular formula of R"-COOH, wherein R" is a saturated or unsaturated C₁-C₅ aliphatic chain. Preferably the R" group is a saturated chain, i.e R" is a substituted or unsubstituted C₁-C₅ alkyl group.

In a particular embodiment, the R" group is an unsubstituted saturated chain, that is R' is an unsubstituted C₁-C₅ alkyl group, preferably an unsubstituted C₁-C₃ alkyl group.

The aliphatic C₂-C₆ monocarboxylic acids can be substituted or unsubstituted. The substituents may be selected from the groups consisting of -OH, -COOH and =O. Preferably, the aliphatic C₂-C₆ monocarboxylic acid has one, two or three substituents selected from -OH, -COOH and =O, more preferably one or two.

In an embodiment, the aliphatic C₂-C₆ monocarboxylic acid is an aliphatic C₂ monocarboxylic acid, aliphatic C₃ monocarboxylic acid or aliphatic C₄ monocarboxylic acid.

In a particular embodiment, the aliphatic C₂-C₆ monocarboxylic acid is selected from substituted or unsubstituted acetic acid and propionic acid. Preferably, it is selected from acetic acid and propionic acid.

One aspect of the invention is directed to a supersaturated aqueous solution of bilastine, comprising an organic carboxylic acid selected from glutaric acid, citric acid, α-cetoglutaric acid, tartaric acid, malic acid, adipic acid, succinic acid, acetic acid, propionic acid and mixtures thereof. In another embodiment, the organic carboxylic acid is selected from glutaric acid, citric acid, tartaric acid, α-cetoglutaric acid, acetic acid, propionic acid and mixtures thereof. In a further embodiment, the organic carboxylic acid is selected from glutaric acid, citric acid, tartaric acid and mixtures thereof. Preferably, the organic carboxylic acid is glutaric acid.

In a particular embodiment, the supersaturated aqueous solution of the invention has a pH between 3 and 6, preferably between 3.5 and 5, more preferably between 4 and 4.5.

In a particular embodiment, the organic carboxylic acid is present in an amount so that the supersaturated aqueous solution has a pH of 3-6, preferably 3.5-5, more preferably 4-4.5.

In a particular embodiment, the molar ratio of bilastine:organic carboxylic acid is from 1:0.2 to 1:3, preferably from 1:0.3 to 1:2.5, more preferably from 1:0.5 to 1:2.

In a particular embodiment, the concentration of bilastine in the supersaturated solution of the invention is at least 1.3 times, preferably at least 1.5 times, more preferably at least 2 times, the concentration of bilastine in the saturated solution.

The concentration of bilastine is at least 2.5 mg/ml, preferably at least 3.5 mg/ml, more preferably at least 4.5 mg/ml, at room temperature.

The concentration of bilastine is at least 2.5 mg/ml at room temperature and a pH value higher than or equal to 4.2.

In another embodiment, the concentration of bilastine is at least 3.5 mg/ml at room temperature and a pH value higher than or equal to 4.2.

In a further embodiment, the concentration of bilastine is at least 2.5 mg/ml at room temperature and a pH between 4.2 and 4.4.

As used herein, "room temperature" or its abbreviation "rt" is taken to mean between 20 to 25 °C. "Standard ambient conditions of temperature and pressure" or "standard ambient conditions" mean a temperature of about 20 to 25 °C and an absolute pressure of about 1 atm.

In a particular embodiment, the supersaturated aqueous solution is substantially free of further solubilizing agents, such as cyclodextrins, water-soluble polymers. The term "substantially free" means that the solution contains less than 1 wt.%, preferably less than 0.5 wt.%, more preferably less than 1000 ppm, of further solubilizing agents.

### Methods of preparation

As mentioned above, it has been observed that organic carboxylic acids as disclosed above allow obtaining stable supersaturated aqueous solutions of bilastine. Therefore, in one embodiment, the invention is directed to the use of an organic carboxylic acid selected from the group consisting of substituted or unsubstituted aliphatic C₃-C₈ α,ω-dicarboxylic acids and substituted or unsubstituted aliphatic C₂-C₆ monocarboxylic acids and mixtures thereof as defined above to increase the aqueous solubility of bilastine as defined above.

In a particular embodiment, the invention is directed to the use of an organic carboxylic acid selected from the group consisting of substituted or unsubstituted aliphatic C₃-C₈ α,ω-dicarboxylic acids and substituted or unsubstituted aliphatic C₂-C₆ monocarboxylic acids and mixtures thereof as defined above to prepare a supersaturated aqueous solution of bilastine as defined above.

In another aspect, the invention is directed to the use of organic carboxylic acid selected from glutaric acid, citric acid, α-cetoglutaric acid, tartaric acid, acetic acid, propionic acid and mixtures thereof as defined above to prepare a supersaturated aqueous solution of bilastine as defined above.

In a particular embodiment, a supersaturated aqueous solution of bilastine as defined herein can be prepared by a process comprising:
(a) preparing a slurry of bilastine and the organic carboxylic acid in water, and
(b) stirring the slurry of step (a) at room temperature.

In a particular embodiment, the slurry of step (a) has a pH between 4.2 and 6, preferably between 4.2 and 5, more preferably between 4.2 and 4.5.

In a particular embodiment, the organic carboxylic acid of step (a) is present in an amount so that the supersaturated aqueous solution has a pH of 4.2-6, preferably 4.2-5, more preferably 4.2-4.5.

In a particular embodiment, the slurry of step (a) comprises a molar ratio of bilastine:organic carboxylic acid from 1:0.2 to 1:3, preferably from 1:0.3 to 1:2.5, more preferably from 1:0.5 to 1:2.

Optionally, the process may include a step of filtration after step (b).

In another aspect, a supersaturated aqueous solution of bilastine as defined herein can be prepared by a process comprising:
(a) preparing a slurry of bilastine and the organic carboxylic acid in water, and
(b) heating the slurry of step (a), and
(c) cooling the resulting composition.

In a particular embodiment, the slurry of step (a) comprises a molar ratio of bilastine:organic carboxylic acid from 1:0.2 to 1:3, preferably from 1:0.3 to 1:2.5, more preferably from 1:0.5 to 1:2.

In yet another particular embodiment, the slurry of step (a) is at a pH between 4.2 and 6, preferably between 4.2 and 5, more preferably between 4.2 and 4.5.

In step (b), the slurry is heated preferably until a solution is obtained. That is until bilastine is completely dissolved. In a particular embodiment, heating in step (b) refers to at least 60 °C, at least 65 °C, at least 70 °C, at least 75 °C, at least 80 °C, at least 85 °C or at least 90 °C. In a particular embodiment, heating in step (b) is between 60 and 100 °C, preferably between 70 and 100°C, more preferably between 80 and 100°C. The composition obtained in step (b) is then cooled to form a supersaturated solution of bilastine. Preferably, it is cooled to room temperature. In a particular embodiment, it is cooled slowly to room temperature, preferably over 2-10 h, more preferably over 3-6 h.

Optionally, the process may include a step of filtration step between step (b) and step (c).

In another aspect of the invention, a supersaturated aqueous solution of bilastine as defined herein can be prepared by a process comprising:
(a) preparing a co-crystal of bilastine and the organic carboxylic acid, and
(b) dissolving the co-crystal of step (a) in water or an aqueous solution.

The co-crystal of step (a) can be prepared by techniques well-known in the art for the preparation of co-crystals. For instance, the co-crystal can be prepared by slurrying and by wet grinding (liquid or solvent assisted grinding).

In an embodiment, the co-crystal can be prepared by a slurrying process comprising:
i) stirring a mixture of bilastine and the organic carboxylic acid in an appropriate solvent, preferably water, at a temperature between room temperature and 40°C;
ii) cooling the mixture to room temperature if temperature of the resulting mixture of the step a) is higher than room temperature, and
iii) isolating the obtained compound.

The step i) may be performed by mixing equimolar amounts of bilastine and the carboxylic acid, and slurrying the mixture in the appropriate solvent. In a preferred embodiment the solvent is water. Step ii) is performed only when the slurry of step i) presents a temperature higher than room temperature. The obtained solid suspended in the solvent is isolated in step iii). The isolation of the solid may include, for example, one or more of the following operations: filtration, filtration under vacuum, evaporation, decantation, and centrifugation and other suitable techniques as known to a skilled person in the art. The cocrystal may be purified, e.g. by recrystallization.

In an embodiment, the co-crystal can be prepared by a liquid assisted grinding process comprising:
i) wet grinding of bilastine and the organic carboxylic acid in an appropriate solvent, preferably water, and
ii) isolating the obtained compound.

The grinding may be performed, for instance, in a ball mill. In a preferred embodiment the solvent is present in catalytic amount. The isolation of the solid may include, for example, one or more of the following operations: filtration, filtration under vacuum, evaporation, decantation, and centrifugation and other suitable techniques as known to a skilled person in the art. The cocrystal may be further purified, e.g. by recrystallization.

"Appropriate solvent" as used herein means a solvent or mixture of solvents selected from the group consisting of water, acetonitrile, dimethylsulfoxide, methanol, ethanol, isopropyl alcohol, ethyl acetate, isobutyl acetate, acetone, methyl isobutyl ketone, tetrahydrofurane, dioxane, diethylether, methyl tert-butyl ether, dichloromethane, chloroform, toluene, cyclohexane, xylene, heptane, dimethylformamide and N-methyl-2-pyrrolidone, preferably water, acetonitrile, methanol, ethanol and chloroform. In a particular embodiment the solvent is water or a mixture of water and other of the above mentioned "appropriate solvents". In another embodiment the solvent is ethanol or a mixture of ethanol and other of the above mentioned "appropriate solvents".

Other processes for obtaining the cocrystals known in the art may be used, such as for example, evaporation, crystallization by cooling, and heating-melting.

The process for preparing a cocrystal comprises putting in contact at least the first neutral component bilastine and a second neutral cocrystal forming compound. Without wishing to be bound by any particular theory, the process is such that it is believed that the first neutral component bilastine can either exist as a neutral zwitterionic species, wherein the acidic moiety is deprotonated and, simultaneously, the pyridinic nitrogen of the benzimidazole moiety is protonated or, as a neutral species, in which both acidic and benzimidazole moieties are neutral. In either case, the net charge of bilastine in the process of the invention is zero and thus the first component of the cocrystal is indeed neutral. Furthermore, the conditions are such that the cocrystal forming component is neutral and thus believed to lack any charges due to the pKa difference between the two cocrystal components. Therefore, the cocrystal forming component does not form ionic interactions with other molecules. As will be apparent to the skilled person, if the solvent is water, the pH is such that there is no deprotonation of the second cocrystal forming compound while if the solvent is an organic solvent, then there are no species responsible for deprotonating the second cocrystal forming compound.

In one embodiment the cocrystal comprises bilastine and glutaric acid. In a particular embodiment the molar ratio of bilastine:glutaric acid is 1:1. In a particular embodiment the molar ratio of bilastine:glutaric acid is 2:1. In another particular embodiment the cocrystal of bilastine and glutaric acid contains water molecules, i.e. it is a hydrate. In an embodiment the molar ratio bilastine:glutaric acid:water in said hydrate is between 2:1:3 and 2:1:1.

In a particular embodiment it is a cocrystal of bilastine:glutaric acid in a 2:1 molar ratio, named GL(I), having a X-ray powder diffraction pattern showing characteristic peaks at a reflection angle [2Θ in degrees] as disclosed in Table 3.1 ± 0.2°.

In a particular embodiment it is a cocrystal of bilastine:glutaric acid in a 2:1 molar ratio, named GL(IV), having a X-ray powder diffraction pattern showing characteristic peaks at a reflection angle [2Θ in degrees] as disclosed in Table 3.3 ± 0.2°.

In a particular embodiment it is a cocrystal of bilastine and glutaric acid in a 1:1 molar ratio, named GL(V), having a X-ray powder diffraction pattern showing characteristic peaks at a reflection angle [2Θ in degrees] as disclosed in Table 3.5 ± 0.2°.

### Pharmaceutical compositions

In another aspect, the invention refers to a pharmaceutical composition comprising as supersaturated aqueous solution of bilastine as defined herein and a pharmaceutically acceptable excipient.

The term "pharmaceutically acceptable excipient" refers to a vehicle, diluent, carrier or adjuvant that is administered with the active ingredient. Such pharmaceutical excipients can be sterile liquids, such as water and oils, including those of petroleum, animal, vegetable, or synthetic origin, such as peanut oil, soybean oil, mineral oil, sesame oil, and similar. Water or saline aqueous solutions and aqueous dextrose and glycerol solutions, particularly for injectable solutions, are preferably used as vehicles. Suitable pharmaceutical vehicles are described in "Remington's Pharmaceutical Sciences" by E.W. Martin, 21st Edition, 2005.

In an embodiment, the pharmaceutical composition is for oral or parenteral administration.

The excipients and auxiliary substances necessary to manufacture the desired pharmaceutical form of administration of the pharmaceutical composition of the invention will depend, among other factors, on the elected administration pharmaceutical form. Said pharmaceutical forms of administration of the pharmaceutical composition will be manufactured according to conventional methods known by the skilled person in the art. A review of different active ingredient administration methods, excipients to be used and processes for producing them can be found in "Tratado de Farmacia Galénica", C. Faulí i Trillo, Luzán 5, S.A. de Ediciones, 1993.

### Uses

Benzimidazole compounds have been found to be antagonists of histamine H1 receptor and are thus useful in the treatment and/or prevention of diseases known to be susceptible to improvement by antagonism of histamine H1 receptor.

Therefore, an aspect of the invention refers to a supersaturated aqueous solution or to a pharmaceutical composition as defined above for use in the treatment and/or prevention of a disorder or disease susceptible to amelioration by antagonism of H1 histamine receptor. Such diseases are, for example, allergic diseases or disorders.

In another aspect, the invention is directed to a supersaturated aqueous solution or to a pharmaceutical composition as defined above for use in the treatment and/or prevention of an allergic disease or disorder. Preferably, an allergic disease or disorder selected from rhinitis, conjunctivitis, rhinoconjunctivitis, dermatitis, urticaria and asthma. Preferably, an allergic disease or disorder selected from rhinitis, conjunctivitis, rhinoconjunctivitis and asthma. More preferably, the allergic disease or disorder is selected from the group consisting of rhinitis, conjunctivitis and rhinoconjunctivitis.

The term "treatment" or "to treat" in the context of this specification means administration of a compound or formulation according to the invention to ameliorate or eliminate the disease or one or more symptoms associated with said disease. "Treatment" also encompasses ameliorating or eliminating the physiological sequelae of the disease. The term "ameliorate" in the context of this invention is understood as meaning any improvement on the situation of the patient treated.

The term "prevention" or "to prevent" in the context of this specification means administration of a compound or formulation according to the invention to reduce the risk of acquiring or developing the disease or one or more symptoms associated with said disease.

The following examples illustrate the invention and should not be considered as limitative of the invention.

### Examples

### Materials and methods

The following materials have been used: bilastine (suministered by FAES Farma), citric acid (Sigma-Aldrich), DL-tartaric acid (Sigma Aldrich), Hydrochloric acid (Panreac).

### First method for determining the solubility

HPLC analyses were performed by duplicate on an Agilent 1100 series apparatus with a XBridge C-18 column at room temperature. 25 µL sample were injected. The mobile phase (isocratic) was set as following: 52%(Methanol : aqueous octylamine 0.01M pH = 7, 65:35) - 42% (Acetonitrile : aqueous octylamine 0.01M pH = 7, 60:40).

### Alternative method for determining the solubility

HPLC-PDA analysis was carried out using a Acquity BEH C18 1.7 µm 100x2.1 mm column at a temperature of 40 °C. The isocratic mobile phase was composed of acetonitrile/methanol/ammonium bicarbonate 10 mM pH 8.7 (20.2 / 24.8 /55.0 %) (% v/v), at a flow rate of 0.6 mL/min. Samples were centrifuged 10 minutes at 4000 rpm at 20°C, and supernatant diluted to aprox. 20-40 µg/mL in water. These diluted samples were kept at 18°C until analysis. Quantization was performed at 220 nm by area comparison against a calibration curve obtained with bilastine working standard.

### Wet Grinding Experiments for the preparation of cocrystals

The grinding experiments were performed in a Retsch MM400 Ball Mill. The mixtures and the milling balls (stainless steel, diameter: 5 mm) were introduced in 9 position milling jars (stainless steel, cell volume: 1.5 mL), the solvent was added to each mixture with a 10 µL microsyringe and the jars were immediately introduced in the clamping device. The samples were then subjected to a 30 min grinding cycle (frequency: 30 s⁻¹).

### Powder X-Rav Diffraction Analysis

Sample preparation: the non-manipulated samples were mounted on a zero-background silicon holder. Data collection: Diffraction measurements were performed at ambient conditions on a PANalytical X'Pert PRO diffractometer with reflection θ-θ geometry, equipped with Cu K-alpha radiation and a PIXcel detector, operated at 45 kV and 40 mA. The samples were allowed to spin at 0.25 rev/s during the data collection. The measurement angular range was 3.0-40.0° (2θ) with a step size of 0.013°. The scanning speed was 0.3283°/s (10.20 s/step). Programs used: data collection with X'Pert Data Collector v 2.2i and treatment with X'Pert HighScore v 2.2cand X'Pert Data Viewer 1.2d.

### DSC-TGA

Thermogravimetric analyses were recorded in a TA SDT Q600. Samples of 5 mg were weighed (using a microscale AE240, Mettler) into 90 µL open alumina crucibles, and were heated at 10 °C/min between 25 and 300 °C, under a nitrogen flow (50 mL/min). Data collection and evaluation was performed with TA Universal Analysis 2000 v 4.7 software.

### Proton Nuclear Magnetic Resonance (¹H-NMR)

Proton nuclear magnetic resonance analyses were recorded in various deuterated solvents, such as dimethylsulfoxide (DMSO-d₆), methanol (MeOH-d₄) and water (D₂0) in a Varian Mercury 400 spectrometer. Spectra were acquired solving 5-10 mg of sample in 0.6 mL of deuterated solvent.

### Solubility of cocrvstals

Solubility of cocrystals was determined by stirring the product in water at room temperature (400 mg of product in 24 ml of water, 60 vol.). The product was previously milled in order to reduce the crystal size effect. A sample is collected and filtered using a sintered funnel (n° 3) periodically (30, 60, 180 min and after overnight). The filtered solid was immediately analyzed by XRPD, while the mother liquors are filtered through a 0.22 um filter. The concentration of bilastine in the solution was determined by the first method for determining the solubility described above by HPLC analysis. The concentration of Bilastine is determined by HPLC area (in some experiments where a high solubility was detected the mother liquor was diluted by a factor 10). This concentration is compared with the bilastine concentration obtained in the same conditions in order to determine a relative solubility.

As used herein, "room temperature" or its abbreviation "rt" is taken to mean between 20 to 25 °C. "Standard ambient conditions of temperature and pressure" or "standard ambient conditions" mean a temperature of about 20 to 25 °C and an absolute pressure of about 1 atm.

### Example 1. General procedure for preparing a supersaturated aqueous solution according to the invention (Method 1)

Method 1. A slurry of bilastine (120 mg) and the organic carboxylic acid (0.5-2.0 eq.) in water (15 ml) was prepared. The mixture was stirred at room temperature (25 °C) and centrifuged. The concentration of bilastine in the solution was determined by the first method for determining the solubility described above by HPLC analysis.

### Example 1.1. Bilastine - glutaric acid

Slurrying bilastine and glutaric acid (0.5 eq.) gave rise to an elevated solubility (41000 area counts) after one night.

When the pH of a slurry of bilastine was adjusted with HCI to the same pH, a solubility of bilastine of only 28000 area counts was obtained after one night.

### Example 2. General procedure for preparing a supersaturated aqueous solution according to the invention (Method 2)

Method 2. A slurry of bilastine (100 mg) and the organic carboxylic acid (amount needed to obtain a pH of 4.0-4.5) in water (10 ml) was prepared. The mixture was stirred at 95 °C for 2 h and filtered. The filtrate was cooled to room temperature and allowed to stand overnight. After centrifugation, the concentration of bilastine in the solution was determined by the alternative method for determining the solubility described above by HPLC-PDA analysis.

### Example 2.1. Bilastine - citric acid

Citric acid was added to a slurry of 100 mg of bilastine in 10 mL of water to get a pH of 4.3 and the mixture was heated at 95 °C for 2 h. Then the solid was filtered off and the filtrate was allowed to stand overnight at room temperature. The solid was separated by centrifugation and the concentration of bilastine was determined by HPLC-PDA.

### Example 2.2. Bilastine - tartaric acid

D,L-Tartaric acid was added to a slurry of 100 mg of bilastine in 10 mL of water to get a pH of 4.3 and the mixture was heated at 95 °C for 2 h. Then the solid was filtered off and the filtrate was allowed to stand overnight at room temperature. The solid was separated by centrifugation and the concentration of bilastine was determined by HPLC-PDA.

### Example 2.3. Bilastine - hydrochloric acid (Comparative)

Hydrochloric acid 3.7% v/v was added to a slurry of 100 mg of bilastine in 10 mL of water to get a pH of 4.3 and the mixture was heated at 95 °C for 2 h. Then the solid was filtered off and the filtrate was allowed to stand overnight at room temperature. The solid was separated by centrifugation and the concentration of bilastine was determined by HPLC-PDA.

Similar results were obtained for citric acid and tartaric acid. A solubility of 2.7 mg/ml of bilastine at pH 4.3 was observed for both citric acid and tartaric acid, which is clearly above the pH-dependent solubility of bilastine shown in Figure 1. Said solubility was maintained after 24 h. In contrast, the use of hydrochloric acid gave rise to a concentration of bilastine in accordance with its pH-dependent solubility shown in Fig. 1.

### Example 3. Preparation of a supersaturated aqueous solution according to the invention from the corresponding cocrystal (Method 3)

### Example 3.1. Bilastine:glutaric acid cocrystal. Crystalline form GL(I)_bilastine/glutaric acid 2:1 cocrystal

The crystalline form, named as GL(I), was obtained by wet grinding of a bilastine: glutaric acid 1:2 mixture in water.

This crystalline form was also obtained by slurrying bilastine and glutaric acid at a stoichiometry ratio of 1:1 or 1:2 in water. After stirring 15 hours, the obtained solid suspended in the water was isolated by filtration, washed with water and dried under vacuum.

This crystalline form was also obtained by seeding with GL(I) a suspension of BLN(I) (1000 mg, 2.157 mmol) and glutaric acid (570.0 mg, 4.314 mmol) in water (10 mL), at rt. After stirring 18 hours at rt, the obtained solid suspended in water was isolated by filtration, washed with water (2 x 1 mL) and dried under vacuum over 18 hours, to yield 974 mg of GL(I) as a white solid (yield 81%).

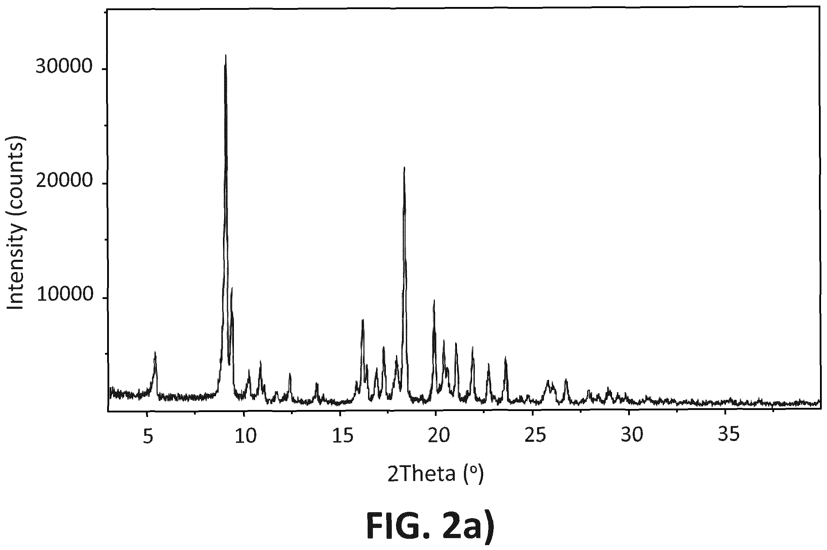

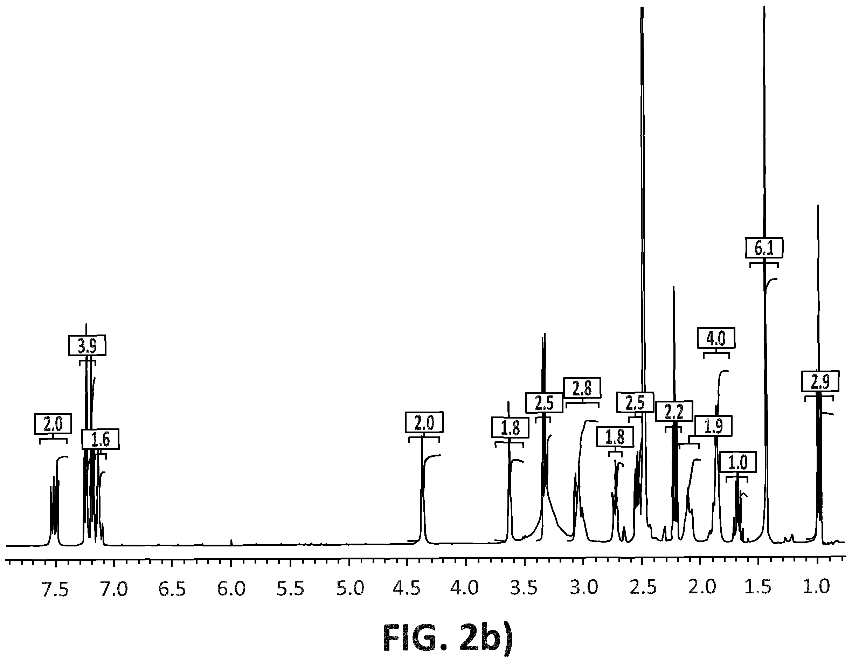

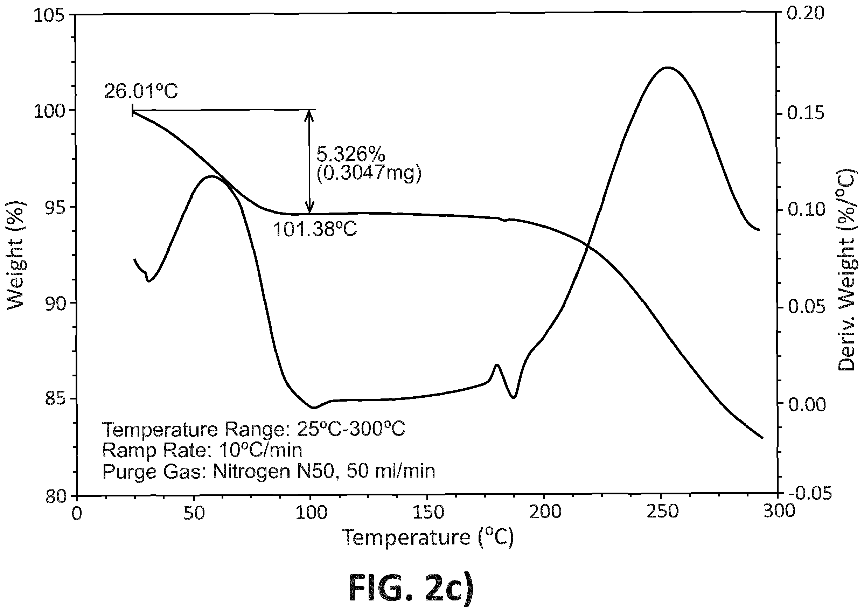

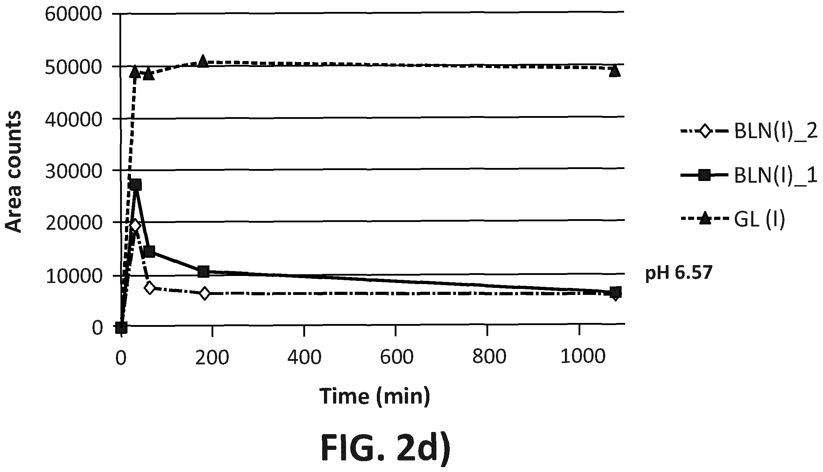

The resulting cocrystal was characterised by XPRD (see Fig. 2 a)), ¹H-NMR (see Fig. 2b)), and TGA (see Fig. 2 c)).

**Table 3.1. XRPD peak list of GL(I):**

| Pos. [°2Th.] | Rel. Int. [%] |
|---|---|
| 5.44 | 12 |
| 9.13 | 100 |
| 9.41 | 32 |
| 10.28 | 8 |
| 10.89 | 12 |
| 11.72 | 2 |
| 12.42 | 8 |
| 13.82 | 5 |
| 15.85 | 4 |
| 16.19 | 22 |
| 16.42 | 9 |
| 16.91 | 9 |
| 17.29 | 15 |
| 17.96 | 11 |
| 18.41 | 67 |
| 19.93 | 27 |
| 20.40 | 17 |
| 20.59 | 9 |
| 21.06 | 17 |
| 21.88 | 15 |
| 22.73 | 10 |
| 23.60 | 12 |
| 24.77 | 2 |
| 25.79 | 6 |
| 26.14 | 3 |
| 26.70 | 7 |
| 27.90 | 4 |
| 28.39 | 1 |
| 28.99 | 3 |
| 29.84 | 2 |
| 31.02 | 2 |
| 35.27 | 1 |

Dissolution of cocrystal GL(I) in water gave rise to a solubility of 48000 area counts (see Fig. 2 d).

**Table 3.2. Solubility data for GL(I):**

| Time | GL (I)_1 | GL (I)_2 | pH_1 | pH_2 |
|---|---|---|---|---|
| 0 min | 0 | 0 | - | - |
| 30 min | 49106 | 48462 | 4.42 | 4.34 |
| 60 min | 48363 | 48871 | 4.31 | 4.3 |
| 180 min | 50828 | 50771 | 4.08 | 4.29 |
| 1080 min | 48315 | 47972 | 4.11 | 3.97 |

### Example 3.2. Bilastine:glutaric acid cocrystal. Crystalline form GL(IV)_bilastine/ glutaric acid 1:1 cocrystal

This crystalline form was obtained by slurrying bilastine and glutaric acid at a stoichiometry ratio of 1:1, 1:2 or 2:1 in ethanol. After stirring for 15 hours, the obtained solid suspended in ethanol was isolated by filtration, washed with ethanol and dried under vacuum.

This crystalline form was also obtained by seeding with GL(IV) a suspension of BLN(I) (1000 mg, 2.157 mmol) and glutaric acid (285.0 mg, 2.157 mmol) in EtOH (10 mL), at rt. After stirring 18 hours at rt, the obtained solid suspended in water was isolated by filtration, washed with water (2 x 1 mL) and dried under vacuum over 18 hours, to yield 1.079 mg of GL(IV) as a white solid (yield 94%).

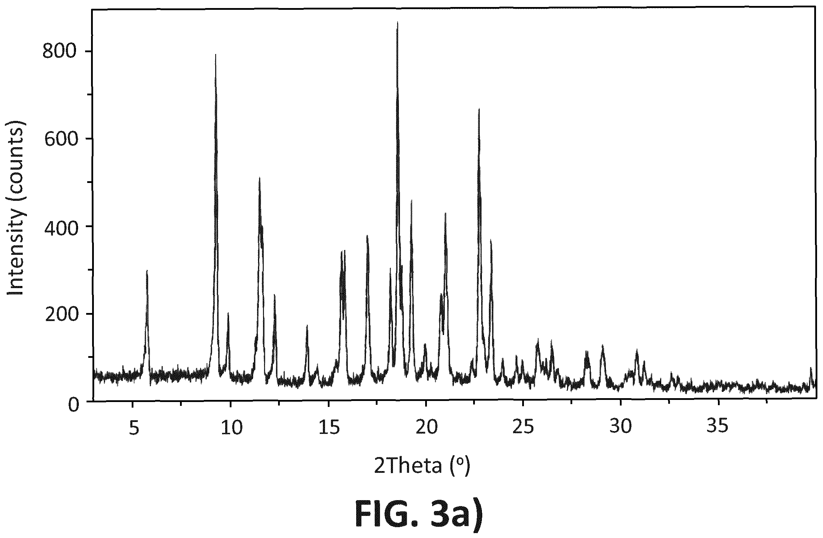

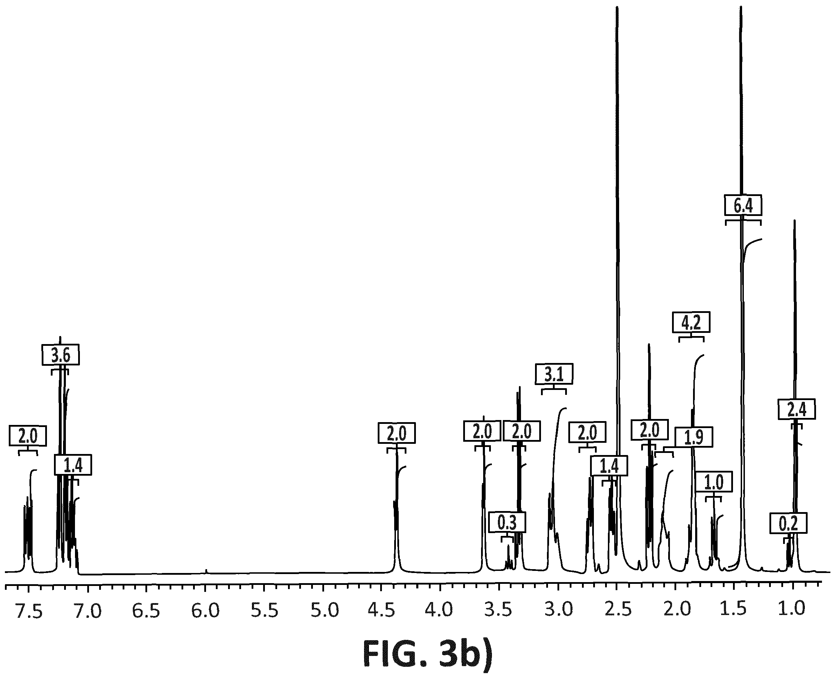

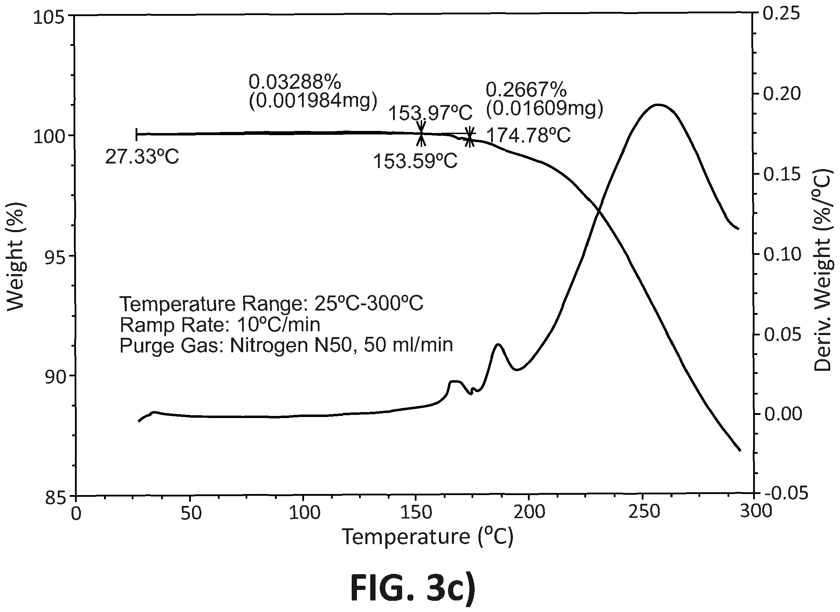

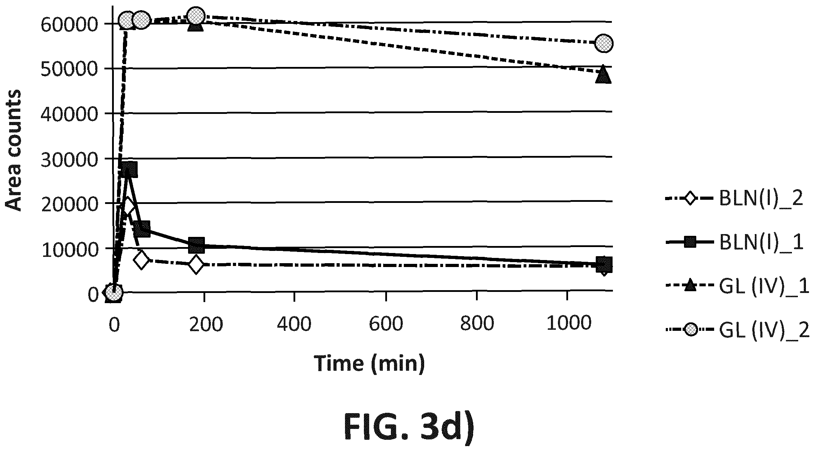

The resulting cocrystal was characterised by XPRD (see Fig. 3 a)), ¹H-NMR (see Fig. 3b)) and TGA (see Fig. 3 c)).

**Table 3.3. XRPD peak list of GL (IV):**

| Pos. [°2Th.] | Rel. Int. [%] |
|---|---|
| 3.18 | 2 |
| 5.85 | 31 |
| 9.39 | 82 |
| 9.97 | 17 |
| 11.61 | 60 |
| 11.73 | 45 |
| 12.33 | 25 |
| 13.98 | 17 |
| 14.50 | 4 |
| 15.45 | 9 |
| 15.73 | 37 |
| 15.93 | 35 |
| 17.10 | 41 |
| 18.29 | 27 |
| 18.67 | 100 |
| 18.85 | 33 |
| 19.36 | 52 |
| 20.03 | 9 |
| 20.86 | 23 |
| 21.08 | 45 |
| 22.40 | 4 |
| 22.82 | 81 |
| 23.42 | 42 |
| 24.00 | 7 |
| 24.70 | 7 |
| 25.01 | 7 |
| 25.77 | 11 |
| 26.22 | 6 |
| 26.51 | 10 |
| 26.78 | 4 |
| 28.21 | 8 |
| 28.37 | 7 |
| 29.08 | 12 |
| 30.80 | 9 |
| 31.21 | 7 |
| 32.60 | 3 |
| 32.95 | 3 |
| 35.93 | 2 |
| 37.84 | 1 |
| 39.75 | 6 |

Dissolution of cocrystal GL(IV) in water gave rise to a solubility of around 60000 area counts as shown in Figure 3.d).

**Table 3.4. Solubility data for GL(IV):**

| Time | GL (IV)_1 | GL (IV)_2 | pH_1 | pH_2 |
|---|---|---|---|---|
| 0 min | 0 | 0 | - | - |
| 30 min | 61110 | 61019 | 4.25 | 4.25 |
| 60 min | 61558 | 61328 | 4.22 | 4.22 |
| 180 min | 60841 | 62159 | 4.06 | 4.11 |
| 1080 min | 48734 | 55535 | 4.00 | 3.98 |

### Example 3.3. Bilastine:glutaric acid cocrystal. Crystalline form GL(V)_bilastine/ glutaric acid 1:1 cocrystal

This crystalline form was obtained by slurrying bilastine and glutaric acid at a stoichiometry ratio of 1:2 in MIK, AcOⁱBu or TBME.

This crystalline form was also obtained by slurrying bilastine and glutaric acid at a stoichiometry ratio of 1:1 or 1:2 in TBME or ACN. After stirring 18 hours, the obtained solid suspended in the solvent was isolated by filtration, washed with the same solvent and dried under vacuum.

This crystalline form was also obtained by seeding with GL(V) a suspension of BLN(I) (1000 mg, 2.157 mmol) and glutaric acid (570.0 mg, 4.314 mmol) in ACN (10 mL), at rt. After stirring 18 hours, the obtained solid suspended in ACN was isolated by filtration, washed with ACN (2 x 1 mL) and dried under vacuum over 18 hours, to yield 1.157 mg of GL(V) as a white solid.

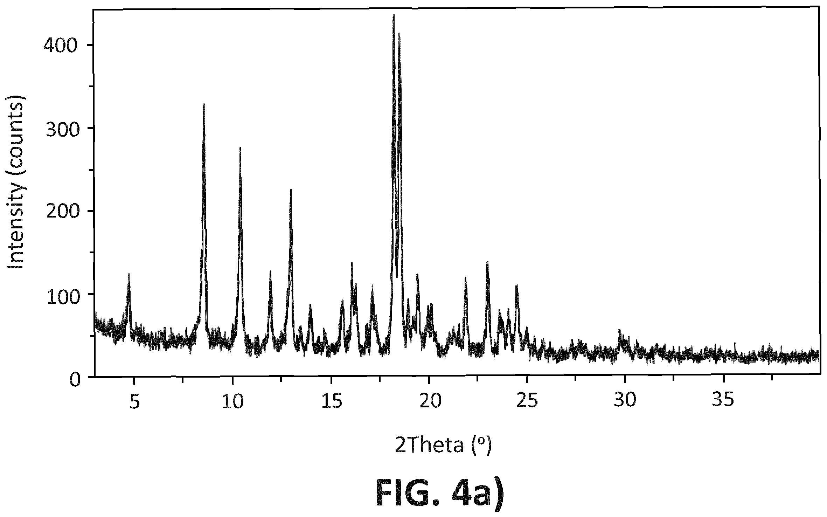

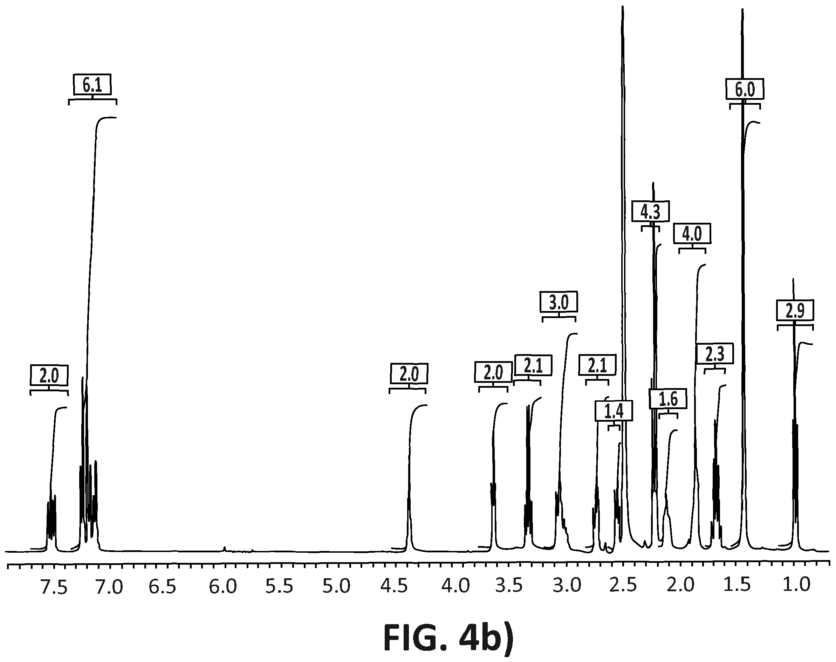

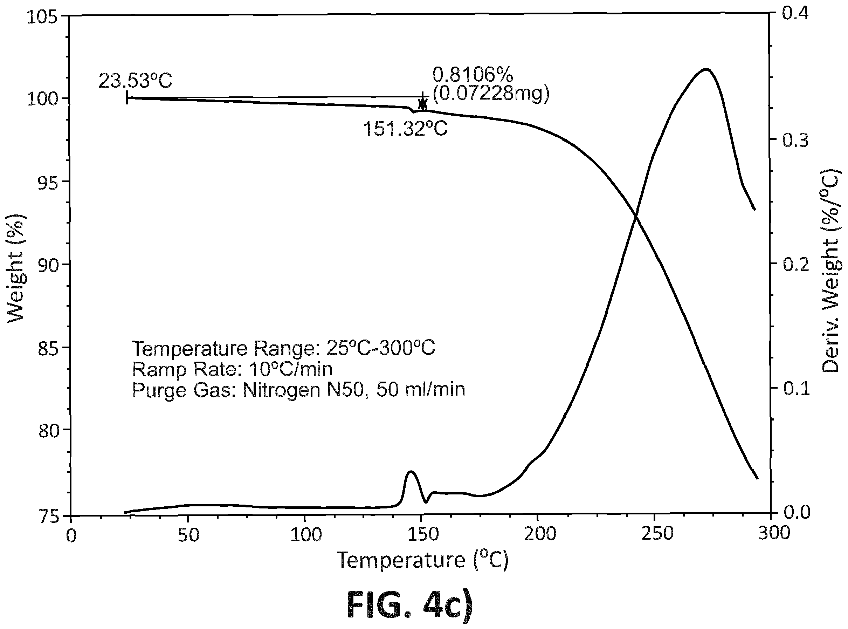

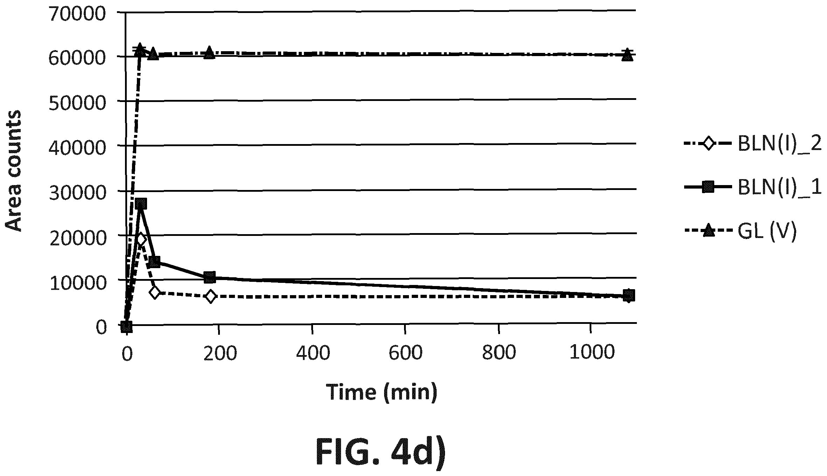

The resulting cocrystal was characterised by XPRD (see Fig. 4 a)), ¹H-NMR (see Fig. 4b)) and TGA (see Fig. 4 c)).

**Table 3.5. XRPD peak list of GL (V):**

| Pos. [°2Th.] | Rel. Int. [%] |
|---|---|
| 4,75 | 18 |
| 8,63 | 76 |
| 10,47 | 64 |
| 12,00 | 22 |
| 13,02 | 46 |
| 13,50 | 6 |
| 14,03 | 12 |
| 14,73 | 5 |
| 15,64 | 15 |
| 16,12 | 24 |
| 16,33 | 19 |
| 16,88 | 7 |
| 17,14 | 17 |
| 18,34 | 96 |
| 18,62 | 100 |
| 19,02 | 14 |
| 19,50 | 23 |
| 19,96 | 9 |
| 20,19 | 12 |
| 21,19 | 4 |
| 21,94 | 22 |
| 23,05 | 27 |
| 23,66 | 12 |
| 24,12 | 11 |
| 24,55 | 21 |
| 25,03 | 7 |
| 25,89 | 4 |
| 27,66 | 2 |
| 29,79 | 7 |
| 30,60 | 3 |
| 31,58 | 3 |

Dissolution of cocrystal GL(V) in water gave rise to a solubility of 60000 area counts (see Fig. 4 d).

**Table 3.6. Solubility data for GL(V):**

| Time | GL (V)_1 | GL (V)_2 | pH_1 | pH_2 |
|---|---|---|---|---|
| 0 min | 0 | 0 | - | - |
| 30 min | 61517 | 62303 | 4.00 | 3.97 |
| 60 min | 60930 | 60916 | 3.99 | 3.95 |
| 180 min | 60628 | 61316 | 3.93 | 3.94 |
| 1080 min | 59755 | 60769 | 3.95 | 3.97 |

## Claims

1. A supersaturated aqueous solution of bilastine, comprising an organic carboxylic acid selected from glutaric acid, citric acid, α-cetoglutaric acid, tartaric acid and mixtures thereof, wherein the concentration of bilastine is at least 2.5 mg/ml at a temperature between 20 to 25 °C and a pH value higher than or equal to 4.2.

2. Aqueous solution according to claim 1, wherein the organic carboxylic acid is glutaric acid.

3. Aqueous solution according to anyone of claims 1 to 2, wherein the concentration of bilastine is at least 1.3 times the concentration of bilastine in the saturated solution.

4. Aqueous solution according to claim 1, wherein the concentration of bilastine is at least 3.5 mg/ml at a temperature between 20 to 25 °C and a pH value higher than or equal to 4.2.

5. Aqueous solution according to claim 1, wherein the concentration of bilastine is at least 2.5 mg/ml at a temperature between 20 to 25 °C and a pH between 4.2 and 4.4.

6. Use of an organic carboxylic acid selected from glutaric acid, citric acid, α-cetoglutaric acid, tartaric acid and mixtures thereof to increase the aqueous solubility of bilastine as defined in the aqueous solution according to claim 1.

7. Method for preparing a supersaturated aqueous solution as defined in anyone of claim 1 to 5 comprising
(a) preparing a slurry of bilastine and the organic carboxylic acid in water at a pH between 4.2 and 6, and
(b) heating the slurry of step (a) to obtain a solution, and
(c) cooling the solution.

8. Method for preparing a supersaturated aqueous solution as defined in anyone of claim 1 to 5 comprising
(a) preparing a co-crystal of bilastine and the organic carboxylic acid, and
(b) dissolving the co-crystal of step (a) in water or an aqueous solution.

9. A pharmaceutical composition comprising a supersaturated aqueous solution as defined in anyone of claim 1 to 5 and at least one pharmaceutically acceptable excipient.

10. A supersaturated aqueous solution as defined in anyone of claim 1 to 5 or a pharmaceutical composition as defined in claim 9 for use as a medicament.

11. A supersaturated aqueous solution as defined in anyone of claim 1 to 5 or a pharmaceutical composition as defined in claim 9 for use in the prevention and/or treatment of an allergic disease or disorder.

12. An aqueous solution for use or pharmaceutical composition for use according to claim 11 wherein the allergic disease or disorder is selected from rhinitis, conjunctivitis, rhinoconjuntivitis, dermatitis, urticaria and asthma.

## Patentansprüche

1. Eine übersättigte wässrige Lösung von Bilastin, umfassend eine organische Carbonsäure, ausgewählt aus Glutarsäure, Zitronensäure, α-Cetoglutarsäure, Weinsäure und Gemischen davon, wobei die Konzentration von Bilastin mindestens 2,5 mg/ml bei einer Temperatur zwischen 20 bis 25°C und einem pH-Wert von 4,2 oder höher beträgt.

2. Wässrige Lösung nach Anspruch 1, wobei die organische Carbonsäure Glutarsäure ist.

3. Wässrige Lösung nach einem der Ansprüche 1 bis 2, wobei die Konzentration von Bilastin mindestens das 1,3-Fache der Konzentration von Bilastin in der gesättigten Lösung beträgt.

4. Wässrige Lösung nach Anspruch 1, wobei die Konzentration von Bilastin mindestens
3,5 mg/ml bei einer Temperatur zwischen 20 bis 25°C und einem pH-Wert von 4,2 oder höher beträgt.

5. Wässrige Lösung nach Anspruch 1, wobei die Konzentration von Bilastin mindestens
2,5 mg/ml bei einer Temperatur zwischen 20 bis 25 °C und einem pH-Wert zwischen 4,2 und 4,4 beträgt.

6. Verwendung einer organischen Carbonsäure, ausgewählt aus Glutarsäure, Zitronensäure, α-Cetoglutarsäure, Weinsäure und Gemischen davon, um die Wasserlöslichkeit von Bilastin wie in der wässrigen Lösung nach Anspruch 1 definiert zu erhöhen.

7. Verfahren zur Herstellung einer übersättigten wässrigen Lösung wie in einem der Ansprüche 1 bis 5 definiert, umfassend
(a) Herstellen einer Aufschlämmung aus Bilastin und der organischen Carbonsäure in Wasser bei einem pH-Wert zwischen 4,2 und 6, und
(b) Erwärmen der Aufschlämmung aus Schritt (a), um eine Lösung zu erhalten, und
(c) Abkühlen der Lösung.

8. Verfahren zur Herstellung einer übersättigten wässrigen Lösung wie in einem der Ansprüche 1 bis 5 definiert, umfassend
(a) Herstellen eines Co-Kristalls aus Bilastin und der organischen Carbonsäure, und
(b) Lösen des Co-Kristalls von Schritt (a) in Wasser oder einer wässrigen Lösung.

9. Ein Arzneimittel, umfassend eine übersättigte wässrige Lösung wie in einem der Ansprüche 1 bis 5 definiert und mindestens einen pharmazeutisch verträglichen Exzipienten.

10. Eine übersättigte wässrige Lösung wie in einem der Ansprüche 1 bis 5 definiert oder ein Arzneimittel wie in Anspruch 9 definiert zur Verwendung als Medikament.

11. Eine übersättigte wässrige Lösung wie in einem der Ansprüche 1 bis 5 definiert oder ein Arzneimittel wie in Anspruch 9 definiert zur Verwendung bei der Vorbeugung und/oder Behandlung einer allergischen Erkrankung oder Störung.

12. Eine wässrige Lösung zur Verwendung oder ein Arzneimittel zur Verwendung nach Anspruch 11, wobei die allergische Erkrankung oder Störung aus Rhinitis, Konjunktivitis, Rhinokonjunktivitis, Dermatitis, Urticaria und Asthma ausgewählt ist.

## Revendications

1. Une solution aqueuse sursaturée de bilastine, comprenant un acide carboxylique organique choisi parmi l'acide glutarique, l'acide citrique, l'acide α-cétoglutarique, l'acide tartrique et les mélanges de ceux-ci, la concentration en bilastine étant d'au moins 2,5 mg/ml à une température comprise entre 20 et 25°C et une valeur pH supérieure ou égale à 4,2.

2. Solution aqueuse selon la revendication 1, dans laquelle l'acide carboxylique organique est l'acide glutarique.

3. Solution aqueuse selon l'une quelconque des revendications 1 à 2, dans laquelle la concentration de bilastine est au moins 1,3 fois la concentration de bilastine dans la solution saturée.

4. Solution aqueuse selon la revendication 1, dans laquelle la concentration de bilastine est d'au moins 3,5 mg/ml à une température comprise entre 20 et 25°C et une valeur de pH supérieure ou égale à 4,2.

5. Solution aqueuse selon la revendication 1, dans laquelle la concentration de bilastine est d'au moins 2,5 mg/ml à une température comprise entre 20 et 25°C et un pH compris entre 4,2 et 4,4.

6. Utilisation d'un acide carboxylique organique choisi parmi l'acide glutarique, l'acide citrique, l'acide α-cétoglutarique, l'acide tartrique et les mélanges de ceux-ci pour augmenter la solubilité aqueuse de la bilastine telle que définie dans la solution aqueuse selon la revendication 1.

7. Procédé de préparation d'une solution aqueuse sursaturée telle que définie dans l'une quelconque des revendications 1 à 5, comprenant
(a) le fait de préparer une suspension de bilastine et l'acide carboxylique organique dans de l'eau à un pH compris entre 4,2 et 6, et
(b) le fait de chauffer la suspension de l'étape (a) pour obtenir une solution, et
(c) le fait de refroidir la solution.

8. Procédé de préparation une solution aqueuse sursaturée telle que définie dans l'une quelconque des revendications 1 à 5, comprenant
(a) le fait de préparer un co-cristal de bilastine et l'acide carboxylique organique, et
(b) le fait de dissoudre le co-cristal de l'étape (a) dans de l'eau ou une solution aqueuse.

9. Une composition pharmaceutique comprenant une solution aqueuse sursaturée telle que définie dans l'une quelconque des revendications 1 à 5 et au moins un excipient pharmaceutiquement acceptable.

10. Une solution aqueuse sursaturée telle que définie dans l'une quelconque des revendications 1 à 5 ou composition pharmaceutique telle que définie dans la revendication 9 pour utilisation en tant que médicament.

11. Une solution aqueuse sursaturée telle que définie dans l'une quelconque des revendications 1 à 5 ou composition pharmaceutique telle que définie dans la revendication 9 pour utilisation dans la prévention et/ou le traitement d'une maladie ou d'une affection allergique.

12. Une solution aqueuse ou composition pharmaceutique pour une utilisation selon la revendication 11, dans laquelle la maladie ou le trouble allergique est l'un parmi la rhinite, la conjonctivite, la rhinoconjuntivite, la dermatite, l'urticaire et l'asthme.
